# EUROPEAN PATENT APPLICATION

(11) **EP 4 763 188 A1**
(43) Date of publication of application: **24.06.2026**
(21) Application number: 24220797.5
(22) Date of filing: 17.12.2024
(51) Int. Cl.: A61K 8/64, A61K 8/9794, A61Q 5/00, A61Q 5/02, A61Q 5/10

(54) **BARLEY PROTEIN EXTRACT AND ITS USE IN HAIRCARE COSMETIC PRODUCTS**

(71) Applicant: Kemon S.P.A., 06016 San Giustino (PG) (IT)
(72) Inventor: Palazzoli, Pietro, SAN GIUSTINO (PG) (IT); Celestini, Sabrina, SAN GIUSTINO (PG) (IT); Nocentini, Benedetta, SAN GIUSTINO (PG) (IT); Nocentini, Giuliano, SAN GIUSTINO (PG) (IT)
(74) Representative: Villa, Livia

(57) **Abstract**

A process for extracting a barley protein concentrate is disclosed, as well as the barley protein concentrate so-obtained. Also, are disclosed haircare products comprising the barley protein concentrate. Said concentrate has shown to be significantly effective in preventing and repairing damage to the hair structure, while making hair stronger and more resistant to breakage. The process of the invention is an ecological extraction process that preserves the beneficial properties of spent barley grains, while providing a concentrate enriched in proteins. This process uses advanced technologies to obtain pure and concentrated extracts, ready to be integrated into various haircare cosmetic formulations, such as creams, lotions, shampoos and masks.

## Description

### FIELD OF THE INVENTION

The present invention relates to a process for extracting a barley protein concentrate, as well as the barley protein concentrate so-obtained. Also, the present invention relates to haircare products comprising the barley protein concentrate. Said concentrate has shown to be significantly effective in preventing and repairing damage to the hair structure, while making hair stronger and more resistant to breakage. The process of the invention is an ecological extraction process that preserves the beneficial properties of spent barley grains, while providing a concentrate enriched in proteins. This process uses advanced technologies to obtain pure and concentrated extracts, ready to be integrated into various haircare cosmetic formulations, such as creams, lotions, shampoos and masks.

### BACKGROUND ART

Plant based protein concentrates produced from oil seeds, such as soybeans or canola, peas, grains such as wheat and corn, distillers' grains (from corn based ethanol production), nuts, etc., are widely used as a protein ingredient in aquaculture feeds, livestock feeds, pet foods and in human foods. Use of plant derived protein concentrates to produce meat substitutes for human food is expanding rapidly.

Currently available plant based protein concentrates have characteristics or compositions (such as ethanol, excess glucose, and allergens) which restrict and limit the quantity or concentration that can be incorporated into finished animal feed or human food products. Those characteristics or compositions further limit use of those protein concentrates in certain applications or raise human health concerns.

Barley has not been used as a feedstock for producing protein concentrates except as described in U.S. Pat. Nos. 8,481,677 and 9,644,228. 8,481,677 claimed the creation of a protein concentrate by an enzymatic, and fermentation process with low temperature drying. U.S. Pat. No. 9,644,228 claimed the creation of a palatable protein requiring fermentation and then an active distillation step after the final separation of the protein solid from the liquids. The process described in these patents separates protein containing solids after fermentation. Those patents include process steps to distil ethanol created by the added organisms such as fungi, bacteria or yeast used to ferment the solubilized grains and oil seeds (including barley) after the final separation of solids and liquids, resulting in the recovery and drying of solids to produce protein concentrate. For the process in those patents the protein concentrate is a direct product of both solubilization and fermentation of carbohydrates in the grain or oilseed. Additionally, the protein concentrate contains more than an insignificant volume of the cell mass of the fermentation organism, as well as fermentation products such as organic acids and the insoluble protein from the grain or oilseed that were not removed in the mandatory distillation step.

The protein concentrate and processes of U.S. Pat. Nos. 8,481,677 and 9,644,228 require integration of fermentation organisms and a fermentation step producing ethanol and requiring culturing of microbes in the liquids to provide cell mass. Therefore, industrial distillation is a mandatory part of those processes. Because the process includes fermentation, the protein concentrate of U.S. Pat. Nos. 8,481,677 and 9,644,228 contains residual ethanol, other fermentation products, residual sugars, cells of the fermentation organisms, and cell mass recovered in the solids. The pH of the protein concentrate from the process of U.S. Pat. Nos. 8,481,677 and 9,644,228 is about 4.0 to 4.2. The protein concentrate in the prior inventions include cell mass of fermentation organisms and have lower protein concentration and increased amounts of residual glucose, other soluble sugars and soluble minerals.

Therefore, the object of the present invention is to provide a high quality, barley protein concentrate with composition and characteristics to overcome limitations of current plant proteins and processes for producing plant protein concentrates, so as to be effectively and safely used in haircare products.

### SUMMARY OF THE INVENTION

The above object has been achieved by a process for extracting a barley protein concentrate, as set forth by the present claims.

In another aspect, the present invention also relates to a barley protein concentrate obtained by the above extraction process.

In a further aspect, the present invention also relates to haircare products comprising said barley protein concentrate.

### BRIEF DRESCTIPTION OF THE DRAWINGS

The characteristics and advantages of the present invention will be evident from the detailed description given below, from the illustrative non-limiting working examples, as well as from the annexed figures, wherein:
Figures 1A and 1B show the images recorded via Motic^{®} Panthera S Light Microscope, at 40x magnification, of human hair treated with comparative products, as per Example 4, and
Figures 2A and 2B show the images recorded via Motic^{®} Panthera S Light Microscope, at 40x magnification, of human hair treated with products comprising the barley protein concentrate of the invention, as per Example 4.

### DETAILED DESCRIPTION OF THE INVENTION

The present invention therefore relates to a process for extracting a barley protein concentrate.

Spent barley grains have a number of appreciable properties, such as:
- Natural Antioxidants: barley grains contain phenolic compounds that counteract free radicals, helping to reduce the signs of aging and protect the skin from environmental damage;
- Hydration and Nourishment: the richness of fibre and protein in barley grains helps keep the skin and hair hydrated and well nourished, improving their elasticity and brightness;
- Soothing Properties: barley grain extracts can soothe irritated skin and reduce inflammation, making them ideal for formulations intended for sensitive or problematic skin. The process of the invention is an ecological extraction process that preserves those beneficial properties of spent barley grains, while providing a concentrate enriched in proteins. This process uses advanced technologies to obtain pure and concentrated extracts, ready to be integrated into various haircare cosmetic formulations, such as creams, lotions, shampoos and masks.

The process for extracting a barley protein concentrate comprises the steps of:
i) providing a water suspension of 2-20wt% spent barley grains,
ii) adjusting the pH of the water suspension to basic, under stirring,
iii) heating the basic suspension to a temperature of 45-75°C and keeping under stirring for at least 1 hour, and
iv) filtering off the suspension and then cooling down the resulting filtrate to 15-25°C, where said filtrate is the barley protein concentrate.

In step i), spent barley grains are provided and then suspended in water to achieve a concentration of 2-20wt%, preferably 5-15wt%.

In preferred embodiments, said spent barley grains is a by-product deriving from local production of beer, so that the process of the invention represents a significant step towards a circular economy, while helping to reduce the overall waste and promoting sustainability. Exploiting these by-products not only reduces the environmental impact of beer production, but also creates added value for the cosmetics industry, that utilizes the resulting barley protein concentrate as active ingredient.

In step ii), the water suspension is stirred, while the pH is adjusted to basic. Preferably, the pH is adjusted to 8-14, by adding an inorganic base, such as sodium hydroxide or potassium hydroxide.

In preferred embodiments, step ii) is performed by applying ultrasonic technology.

In step iii), the basic suspension is heated to a temperature of 45-75°C, then kept under stirring for at least 1 hour. Preferably, the basic suspension is heated to a temperature of 55-65°C, then preferably under stirring for 1-8 hours.

In preferred embodiments, step iii) is performed by applying ultrasonic technology.

In step iv), the suspension is filtered off and the resulting filtrate is then cooled down to 15-25°C, where said filtrate is the barley protein concentrate of the invention.

In preferred embodiments, at the end of step iv), the barley protein concentrate is added with a preservative agent, preferably in a concentration of 0.1-5wt%, based on the concentrate weight. Preferably, said preservative agent comprises potassium sorbate, sodium benzoate, benzyl alcohol, phenoxyethanol, caprylyl glycol, or a mixture thereof.

In preferred embodiments, at the end of step iv), the barley protein concentrate is added with a rheological additive, preferably in a concentration of 0.1-5wt%, based on the concentrate weight. Preferably, said rheological additive comprises xanthan gum, alginates, Arabic gum, tragacanth gum, hydroxyethyl cellulose, methyl cellulose, methyl hydroxypropyl cellulose, sodium carboxymethyl cellulose, *Caesalpinia Spinosa* Gum, hydroxypropyl starch phosphate, or a mixture thereof.

In preferred embodiments, at the end of step iv), the barley protein concentrate is added with an acidifier, preferably in a concentration of 0.01-0.5wt%, based on the concentrate weight, so as to adjust the pH of the barley protein concentrate to 3.5-6.5. Preferably, said acidifier comprises citric acid, ascorbic acid, adipic acid, acetic acid, tartaric acid, tannic acid, lactic acid, glycolic acid or a mixture thereof.

In more preferred embodiments, the barley protein concentrate is added with a preservative agent, a rheological additive, an acidifier, or a mixture thereof.

Optionally, the so-obtained barley protein concentrate can be dried to give a solid product, preferably a powdered product, for instance via freeze-drying or spray drying techniques.

In another aspect, the present invention thus relates to a barley protein concentrate obtained by the above extraction process.

Particularly, the barley protein concentrate obtained by the process of the invention has a protein content of 0.01-2.0 g/100 g of the concentrate. The protein content is preferably measured according to AOAC 992.23-1992(1998) "Crude protein in cereal grains and oilse".

Preferably, the barley protein concentrate obtained by the process of the invention has a protein content of 0.1-1.0 g/100 g of the concentrate.

More preferably, the barley protein concentrate obtained by the process of the invention has a protein content of 0. 1-0.5 g/100 g of the concentrate.

Preferably, the barley protein concentrate obtained by the process of the invention further has a Total Kjeldahl Nitrogen (or TKN) content of 0.001-1.0 g/100 g of the concentrate.

More preferably, the barley protein concentrate obtained by the process of the invention further has a Total Kjeldahl Nitrogen (or TKN) content of 0.01-0.1 g/100 g of the concentrate.

Preferably, the barley protein concentrate obtained by the process of the invention further has an amino acid content of 0.01-2.0 g/100 g of the concentrate. The amino acid content is preferably measured according to ISO 13903:2005 - Determination of amino acids content.

More preferably, the barley protein concentrate obtained by the process of the invention further has an amino acid content of 0.1-1.0 g/100 g of the concentrate.

In preferred embodiments, the amino acid present in the barley protein concentrate is Aspartic acid, Glutamic acid, Alanine, Arginine, Phenylalanine, Leucine, Proline, Serine, Valine, or a mixture thereof.

In a further aspect, the present invention also relates to haircare products comprising said barley protein concentrate.

Preferably, said haircare products comprise 0.1-20wt% barley protein concentrate, based on the haircare product weight.

Suitable haircare products are in a form of lotion, milk, mousse, gel, cream, shampoo, conditioner, compress, mask, oil, emulsion o/w and emulsion w/o, silicone emulsion, multiple emulsion, microemulsion, hydroalcoholic solution, hydroglyceric solution, ointment, lipogel, paste, stick, cream-gel, spray, oxidative dyes, serum, decolouring base, or combination thereof, such as a kit of combined products.

Said haircare products further contain cosmetically acceptable excipients.

Cosmetically acceptable excipients comprise water, preservatives, antioxidants, chelating agents, sunscreening agents, vitamins, silanes, silanols, hair dye agents, keratin softeners, proteins, diluents, amino acids, natural plant extracts, wetting agents, fragrances, perfumes, oils, emollients, lubricants, butters, permeation enhancers, thickeners, viscosity modifiers, polymers, resins, hair binders, film forming agents, surfactants, detergents, emulsifiers, dulling agents, propellants, conditioning agents, liquid vehicles, salts, pH regulators, neutralizing agents, buffers, antistatic agents, anti-frizzing agents, anti-dandruff agents, absorbent agents, and mixtures thereof.

Suitable keratin softeners comprise allantoin, glycols and polyalcohols, such as glycerol, erythritol, sorbitol, pyrrolidone carboxylic acid and salts thereof, betaine, and combinations thereof.

Surfactants can be amphoteric, anionic or cationic.

Suitable surfactants are 3-aminosulfonic acid, almond amide, almond amidopropyl betaine, almond amidopropylamine oxide, aluminium lanolate, aminoethyl sulfate, aminopropyl lauryl glutamine, ammonium C12-15 alkyl sulfate, ammonium C12-15 pareth sulfate, ammonium C12-C16 alkyl sulfate, ammonium C9-10 perfluoroalkyl sulphonate, ammonium caprileth sulfate, ammonium capryleth-3 sulphate, ammonium monoglyceride sulphate, ammonium sulphate, ammonium isothionate, ammonium cocoyl sarcosinate, ammonium cumene sulfonate, ammonium dimethicone copolyol sulphate, ammonium dodecylbenzenesulfonate, ammonium isostearate, ammonium laureth sulfate, ammonium laureth-12 sulfate, ammonium laureth-5 sulfate, ammonium laureth-6 carboxylate, ammonium laureth-7 sulfate, ammonium laureth-8 carboxylate, ammonium laureth-9 sulfate, ammonium lauroyl sarcosinate, ammonium lauryl sulfate, ammonium lauryl sulfosuccinate, ammonium myreth sulfate, ammonium myristyl sulfate, ammonium nonoxynol-30 sulfate, ammonium nonoxinol-4 sulphate, ammonium oleate, ammonium polyacrylate, ammonium stearate, ammonium tallate, ammonium xylene sulfonate, AMP-isostearoyl gelatine/keratin amino acids/lysine hydroxypropyltriammonium chloride, AMP-isostearoyl collagen hydrolysed, PEG-6 esters of apricot kernel oil, apricot amine, apricot amidopropyl betaine, arachideth-20, avocadamide, avocadamido propyl betaine, babassuamide, babassuamidopropyl betaine, babassuamidopropylamine oxide, behenalconium chloride, behenamide, behenamidopropyl betaine, behenamine oxide, sodium laureth sulfate, sodium lauryl sulphate, or combinations thereof.

Suitable anionic surfactants include those containing carboxylated, sulfonated and sulphated ions. Examples of anionic surfactants include sodium, potassium, ammonium of long chain alkyl sulfonates and alkyl aryl sulfonates, such as sodium dodecylbenzene sulfonate; dialkyl sodium sulfosuccinates, such as sodium dodecylbenzene sulfonate; sodium dialkyl sulfosuccinates, such as sodium bis-(2-ethylthioxyl)sulfosuccinate; and alkyl sulphates such as sodium lauryl sulfate. Cationic surfactants include quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, acylated sorbitan, acylated sucrose, laurate PEG-150, monolaurate PEG-400, monolaurate polyoxyethylene, polysorbates, polyoxyethylene octylphenylether, PEG-1000 cetyl ether, polyoxyethylene tridecyl ether, polypropylene glycol butyl ether, Poloxamer 401, and stearoyl monoisopropanolamide. Examples of amphoteric surfactants include sodium N-dodecyl-beta-alanine, sodium N-lauryl-p-iminodipropionate, myristoamphoacetate, lauryl betaine and lauryl sulfobetaine.

The haircare products of the invention optionally and preferably comprise 0.1-15% wt% of surfactants on the haircare products' weight.

Suitable emollients comprise silicones, such as dimethicone, cyclomethicone, dimethicone copolyol or a mixture of cyclopentasiloxane and dimethicone/vinyl dimethicone crosslinked polymer, cyclopentasiloxane polysilicone; polyols such as sorbitol, glycerol, propylene glycol, ethylene glycol, polyethylene glycol, caprylyl glycol, polypropylene glycol, 1,3-butanediol, hexylene glycol, isoprene glycol, xylitol, ethylhexyl palmitate; a triglyceride, such as caprylic/capric triglyceride; and fatty acid esters, such as cetearyl isononanoate or cetyl palmitate.

The haircare products of the invention optionally and preferably comprise 0.1-50% wt% of emollients on the haircare products' weight.

Suitable emulsifiers comprise copolymers of an unsaturated ester and monomeric styrene sulfonate, cetearyl alcohol, glyceryl ester, polyoxyethylene glycol ethers of cetearyl alcohol, stearic acid, polysorbate-20, ceteareth-20, lecithin, stearate glycol, polysorbate-60, or polysorbate-80, and combinations thereof.

The haircare products of the invention optionally and preferably comprise 0.1-10% wt% of emulsifiers on the haircare products' weight.

Suitable preservatives comprise glycerine-containing compounds (for example, glycerine or ethylhexyl glycerol or phenoxyethanol), benzyl alcohol, parabens (methylparaben, ethylparaben, propylparaben, butylparaben, isobutylparabene, etc.), sodium benzoate, Leuconostoc/Radish root ferment filtrate, EDTA, potassium sorbate, grapefruit seed extract, salicylic acid, DMDM hydantoin, imidazolidinyl urea, diazolidinyl urea, methylisothiazolinone, sodium dehydroacetate, dehydroacetic acid, Quaternium-15, stearalconium chloride, zinc pyrithione, sodium metabisulphite, 2-bromo-2-nitropropane, chlorhexidine digluconate, polyaminopropyl biguanide, benzalkonium chloride, sodium sulphite, sodium salicylate, citric acid, neem oil, essential oils, lactic acid, vitamin E, and combinations thereof.

The haircare products of the invention optionally and preferably comprise 0.01 - 5% wt% of preservatives on the haircare products' weight.

In some embodiments, the topical cosmetic composition of the invention does not comprise preservatives.

Suitable conditioning agents include silicones (for example silica Quaternium-8), panthenol, hydrolysed wheat and/or soy proteins, hydrolysed pea protein, amino acids (e.g., wheat amino acids), rice bran wax, mango seed oil, grape seed oil, jojoba seed oil, sweet almond oil, aloe leaf extract, aloe barbadensis leaf juice, phytantriol, retinyl palmitate, behentrimonium methosulfate, cyclopentasiloxane, Quatemium-91, stearamidopropyl dimethylamine, and combinations thereof.

The haircare products of the invention optionally and preferably comprise 0.1-15% wt% of conditioning agents on the haircare products' weight.

As for diluents, water is the preferred diluent, but alcohols such as ethyl alcohol and isopropyl alcohol are also suitable.

Suitable viscosity modifiers are viscous liquids such as polyethylene glycol, semisynthetic polymers, for example semi-synthetic cellulose derivatives, synthetic polymers such as carbomers, polyoxamers and polyethyleneimines, natural polymers such as acacia, tragacanth, alginates (e.g., sodium alginate), carrageenan, vegetable gums, such as xanthan gum, vaseline, waxes, bentonite, colloidal silicon dioxide, and microcrystalline cellulose, salts such as sodium chloride, and combinations thereof.

Suitable antioxidants comprise tocopherols, BHT, ascorbic acid, Camellia sinensis leaf extract, Selaginella lepidophylla extract, ascorbyl palmitate, magnesium ascorbyl phosphate, carotenoids, resveratrol, triethyl citrate, arbutin, cogic acid, tetrahexyldecyl ascorbate, superoxide dismutase, zinc, sodium metabisulphite, ubiquinone, and combinations thereof. Suitable dulling agents comprise glycol distearate and ethoxylated fatty alcohols.

The following haircare products including the barley protein concentrate (shortly "BPC") of the invention, are embodiments particularly preferred:

### a) Shampoos comprising:

| **INCI** | **wt%** | **function** |
|---|---|---|
| AQUA | 70-90% | solvent |
| POLYQUATERNIUM-10 | 0.1-2% | conditioner |
| SODIUM C14-16 OLEFIN SULFONATE | 1-8% | surfactant |
| SODIUM COCOAMPHOACETATE | 1-8% | surfactant |
| LAURETH-5 CARBOXYLIC ACID | 1-8% | surfactant |
| PEG-18 GLYCERYL OLEATE/COCOATE | 1-5% | surfactant |
| PHENOXYETHANOL | 0.1-1% | preservative |
| BENZYL ALCOHOL | 0.1-1% | preservative |
| ETHYLHEXYLGLYCERIN | 0.1-1% | preservative |
| SODIUM GLUCEPTATE | 0.01-0.5% | chelating agent |
| **BPC** | 0.1-20% | active ingredient |
| PARFUM | 0.1-1% | perfume |
| PEG-40 HYDROGENATED CASTOR OIL | 0.1-1% | solvent |
| ETHOXYDIGLYCOL | 0.1-1% | solvent |
| CITRIC ACID | 0.1-1% | pH adjuster |
| PEG-120 METHYL GLUCOSE DIOLEATE | 0.01-1% | thickener |

### b) Conditioners comprising:

| **INCI** | **wt%** | **function** |
|---|---|---|
| AQUA | 60-90% | solvent |
| POLYQUATERNIUM-10 | 0.1-1% | conditioner |
| HYDROXYPROPYL STARCH PHOSPHATE | 0.1-4% | thickener |
| AQUA - CETRIMONIUM CHLORIDE | 0.1-4% | conditioner |
| DISTEAROYLETHYL HYDROXYETHYLMONIUM METHOSULFATE | 0.2-3% | conditioner |
| OLEA EUROPAEA OIL UNSAPONIFIABLES | 0.2-3% | emollient |
| OLEA EUROPAEA FRUIT OIL | 0.2-3% | emollient |
| HYDROGENATED VEGETABLE OIL | 0.2-3% | emollient |
| SORBITAN OLIVATE | 0.2-3% | emulsifier |
| CETEARYL OLIVATE | 0.2-3% | emollient |
| BUTYROSPERMUM PARKII BUTTER | 0.2-3% | emollient |
| CETEARYL ALCOHOL | 0.2-3% | emollient |
| DICOCOYLETHYL HYDROXYETHYLMONIUM METHOSULFATE | 0.2-3% | conditioner |
| PROPYLENE GLYCOL | 0.2-3% | humectant |
| BEHENYL ALCOHOL | 0.2-3% | emollient |
| GUAR HYDROXYPROPYLTRIMONIUM CHLORIDE | 0.2-3% | conditioner |
| HYDROXYPROPYLTRIMONIUM HYDROLYZED WHEAT PROTEIN | 0.2-3% | conditioner |
| SODIUM PCA | 0.2-3% | humectant |
| GLYCERIN | 0.2-3% | humectant |
| **BPC** | 0.1-20% | active ingredient |
| BETAINE | 0.2-3% | humectant |
| POLYQUATERNIUM-47 | 0.2-3% | conditioner |
| AMODIMETHICONE | 0.2-3% | antistatic |
| POTASSIUM SORBATE | 0.1-1% | preservative |
| BENZYL ALCOHOL CONS | 0.1-1% | preservative |
| SODIUM BENZOATE | 0.1-1% | preservative |
| SODIUM GLUCEPTATE | 0.1-1% | chelating agent |
| CITRIC ACID | 0.1-1% | pH adjuster |
| PARFUM | 0.1-1% | perfume |

### c) Masks comprising:

| **INCI** | **wt%** | **function** |
|---|---|---|
| AQUA | 60-90% | solvent |
| BEHENTRIMONIUM CHLORIDE | 0.2-6% | emulsifier |
| BUTYROSPERMUM PARKII BUTTER | 0.2-2% | emollient |
| GLYCERYL LINOLENATE | 0.2-2% | emollient |
| GLYCERYL LINOLEATE | 0.2-2% | emollient |
| GLYCERYL OLEATE | 0.2-2% | emollient |
| CETEARYL ALCOHOL | 2-8% | emollient |
| CETYL ESTERS | 2-5% | emollient |
| QUATERNIUM-87 | 0.5-5% | conditioner |
| PROPYLENE GLYCOL | 0.5-5% | humectant |
| AMODIMETHICONE | 0.5-5% | antistatic |
| ISOSTEARYL NEOPENTANOATE | 0.5-5% | emollient |
| POTASSIUM SORBATE | 0.1-1% | preservative |
| BENZYL ALCOHOL | 0.1-1% | preservative |
| SODIUM BENZOATE | 0.1-1% | preservative |
| SODIUM GLUCEPTATE | 0.1-1% | chelating agent |
| **BPC** | 0.1-20% | active ingredient |
| CITRIC ACID | 0.1-1% | pH adjuster |
| PARFUM | 0.1-1% | perfume |

### d) Leave-in creams comprising:

| **INCI** | **wt%** | **function** |
|---|---|---|
| AQUA | 60-90% | solvent |
| CITRIC ACID | 0.1-1% | pH adjuster |
| CETRIMONIUM CHLORIDE | 0.1-5% | emulsifier |
| CETYL ALCOHOL | 0.1-5% | emollient |
| OLETH-10 | 0.1-5% | emulsifier |
| SORBITAN OLEATE | 0.1-5% | emulsifier |
| DICOCOYLETHYL HYDROXYETHYLMONIUM METHOSULFATE | 0.1-5% | conditioner |
| PROPYLENE GLYCOL | 0.1-5% | humectant |
| DICAPRYLYL ETHER | 0.1-5% | emollient |
| CETEARYL ALCOHOL | 0.1-5% | emollient |
| BUTYROSPERMUM PARKII BUTTER | 0.1-5% | emollient |
| DIMETHICONE | 0.1-5% | emollient |
| PEG/PPG-14/4 DIMETHICONE | 0.1-5% | emulsifier |
| SORBITOL | 0.1-5% | humectant |
| BENZYL ALCOHOL CONS | 0.1-5% | preservative |
| SODIUM BENZOATE | 0.1-5% | preservative |
| POTASSIUM SORBATE | 0.1-5% | preservative |
| SODIUM GLUCEPTATE | 0.1-5% | chelating agent |
| **BPC** | 0.1-20% | active ingredient |
| AMODIMETHICONE | 0.1-5% | antistatic |
| PARFUM | 0.1-5% | perfume |

### e) Hairsprays comprising:

| **INCI** | **wt%** | **function** |
|---|---|---|
| ALCOHOL DENAT. | 50-90% | solvent |
| AMINOMETHYL PROPANOL | 0.01-0.1% | pH adjuster |
| ACRYLATES COPOLYMER | 0.2-15 % | fixative |
| AQUA | 0.1-10 % | solvent |
| **BPC** | 0.1-20%% | active ingredient |
| PARFUM | 0.1 - 0.9 | perfume |

### f) Oxidative hair dyes comprising:

| **INCI** | **wt%** | **function** |
|---|---|---|
| SORBITAN STEARATE | 0.5 - 5% | emulsifier |
| GLYCERYL ISOSTEARATE | 0.5 - 5% | emulsifier |
| COCOS NUCIFERA OIL | 0.5 - 5% | emollient |
| POLYSORBATE 60 | 0.5 - 9% | emulsifier |
| LAURETH-3 | 0.5 - 9% | emulsifier |
| PETROLATUM | 0.5 - 9% | emollient |
| OLEIC ACID | 0.5 - 9% | emulsifier |
| CERA ALBA | 0.5 - 9% | emollient |
| CETEARYL ALCOHOL | 1.5 - 15% | emollient |
| SORBITAN OLEATE | 0.2 - 2% | emulsifier |
| HYDROGENATED CASTOR OIL | 0.2 - 2% | emulsifier |
| CETEARETH-20 | 0.2 - 2% | surfactant |
| STEARIC ACID | 0.2 - 2% | emulsifier |
| PALMITIC ACID | 0.2 - 2% | emulsifier |
| POLYQUATERNIUM-22 | 0.1 - 2% | antistatic |
| ETIDRONIC ACID | 0.01 - 0.5% | chelating agent |
| PROPYLENE GLYCOL | 0.5 - 5% | emollient |
| TETRASODIUM EDTA | 0.01 - 0.5% | chelating agent |
| COCAMIDOPROPYL BETAINE | 0.5 - 6% | surfactant |
| AMMONIA (in combination or substituted with: ETHANOLAMINE, AMINOMETHYL PROPANOL, AMMONIUM BICARBONATE, DIMETHYLGLUCAMINE) | 0.5 - 10% | pH adjuster |
| **BPC** | 0.1-20% | active ingredient |
| AQUA | 60 - 80% | solvent |
| PARFUM | 0.1 - 0.9% | perfume |
| TOLUENE-2.5-DIAMINE SULFATE | 0.01 - 1% | colorant |
| p-AMINOPHENOL | 0.01 - 1% | colorant |
| p-METHYLAMINOPHENOL SULFATE | 0.01 - 1% | colorant |
| m-AMINOPHENOL | 0.01 - 1% | colorant |
| 1-NAPHTHOL | 0.01 - 1% | colorant |
| 4-CHLORORESORCINOL | 0.01 - 1% | colorant |
| 4-AMINO-2-HYDROXYTOLUENE | 0.01 - 1% | colorant |
| 2-METHYLRESORCINOL | 0.01 - 1% | colorant |
| PHENYL METHYL PYRAZOLONE | 0.01 - 1% | colorant |
| SODIUM HYDROSULFITE | 0.01 - 1% | antioxidant |
| ERYTHORBIC ACID | 0.01 - 1% | antioxidant |
| SODIUM SULFITE | 0.01 - 1% | antioxidant |

### g) Decoloring bases comprising:

| **INCI** | **wt%** | **function** |
|---|---|---|
| SODIUM SILICATE | 20-50% | pH adjuster |
| AMMONIUM PERSULFATE | 20-50% | oxidant |
| POTASSIUM PERSULFATE | 13-20% | oxidant |
| SODIUM METASILICATE | 0.2-5% | chelating agent |
| SODIUM STEARATE | 0.2-5% | surfactant |
| CYAMOPSIS TETRAGONOLOBA GUM | 0.2-5% | ligand |
| SODIUM LAURYL SULFATE | 0.2-5% | surfactant |
| MAGNESIUM OXIDE | 0.2-5% | pH adjuster |
| CYCLODEXTRIN | 0.2-5% | chelating agent |
| MAGNESIUM STEARATE | 0.2-5% | antiagglomerant |
| PARAFFINUM LIQUIDUM | 0.2-5% | emollient |
| XANTHAN GUM | 0.2-5% | ligand |
| **BPC** | 0.1-20% | active ingredient |

### h) Serums comprising:

| **INCI** | **%** | **function** |
|---|---|---|
| **BPC** | 0.1-20% | active ingredient |
| AQUA | 60-90% | solvent |
| HYDROXYPROPYL GUAR | 0.1-1% | thickener |
| GLICERINE | 0.1-10% | emollient |
| PROPANEDIOL | 0.1-10% | emollient |
| GUAR HYDROXYPROPYLTRIMONIUM CHLORIDE | 0.1-1% | conditioner |
| BENZOIC ACID | 0.1-1% | preservative |
| DEHYDROACETIC ACID | 0.1-1% | preservative |
| PHENOXYETHANOL | 0.1-1% | preservative |
| SODIUM BENZOATE | 0.1-1% | preservative |
| POTASSIUM SORBATE | 0.1-1% | preservative |
| BENZYL ALCOHOL | 0.1-1% | preservative |
| CETRIMONIUM CHLORIDE | 0.1-1% | surfactant |
| PARFUM | 0.1-0.9% | perfume |

It has been observed that the use of the haircare products comprising the barley protein concentrate of the invention has a significantly appreciable efficacy in hair reparation, as the cuticles appear to be homogenously closed.

Also, the hair treated with the haircare products of the invention have surprisingly shown highly significant decreases in terms of hair brokage and damage, with respect to hair treated with the same haircare products but not including the barley protein concentrate of the invention. As a matter of fact, by comparing the experimental data, an average hair breakage reduction value of more than 65 times was obtained.

This means that the addition of BPC of the invention to haircare products is significantly effective in preventing and repairing damage to the hair structure, while making hair stronger and more resistant to breakage.

The use of barley protein concentrate of the invention in cosmetics represents a significant step towards a circular economy, helping to reduce overall waste and promoting sustainability. Exploiting these by-products not only reduces the environmental impact of beer production, but also creates added value for the cosmetics industry.

The instant invention aims to revolutionize the approach to natural and sustainable cosmetics, by exploiting the potential of barley spent grains to develop innovative products that respect both consumers and environment. By adopting this invention, the cosmetics industry can obtain effective and ecosystem-friendly formulas, thus opening new frontiers in beauty care, and specially haircare.

It is to be understood that all aspects identified as preferred and advantageous for the barley protein concentrate of the invention, are to be deemed analogously preferred and advantageous also for the haircare products, and both the uses and methods of application of the same.

It is also to be understood that all the combinations of preferred and advantageous features of the barley protein concentrate, haircare products, uses and methods are deemed to be hereby described.

Working examples of the present invention provided for illustrative purposes are reported herein below.

### EXAMPLES

### Example 1.

Spent barley grains were suspended in demineralized water at a weight percentage of 10wt%. The suspension was stirred by mechanical or flow processes. A sodium hydroxide solution was added until a basic pH (8-14) is reached. The suspension was heated to a temperature of 60°C and left stirring for 4 hours. The suspension was filtered off, and then cooled down to 20°C (room temperature).

The resulting filtrate was the barley protein concentrate.

A preservative agent was added, comprising potassium sorbate (2wt%), sodium benzoate (1wt%), benzyl alcohol cons (0.5wt%), phenoxyethanol (0.6wt%), caprylyl glycol (3wt%). 3wt% of xanthan gum and 0.2wt% citric acid were then added.

The final composition comprising the barley protein concentrate had a pH of 5.0. Alternatively, the resulting filtrate can also be subjected to freeze-drying or spray drying so as to obtained a barley protein powder.

### Example 2.

The barley protein concentrate obtained in Example 1 was analysed in order to characterize the components present therein.

The protein content was measured according to AOAC 992.23-1992(1998) "Crude protein in cereal grains and oilse".

The amino acid content was measured according to ISO 13903:2005 - Determination of amino acids content.

In the table below, the result of said analysis is reported, in terms of protein content, as well as total nitrogen and amino acid content:

| | **Concentration** | |
|---|---|---|
| Proteins | 0.230±0.018 | g/100 g |
| Total nitrogen (Kjeldahl) | 0.04±0.01 | g/100 g |
| Amino acid content | 0.1804±0.0116 | g/100 g |

Among the amino acids present in the concentrate, the following were detected:

| | | |
|---|---|---|
| Aspartic acid | 0.0178±0.0029 | g/100 g |
| Glutamic acid | 0.0563±0.0090 | g/100 g |
| Alanine | 0.0135±0.0022 | g/100 g |
| Arginine | 0.0138±0.0022 | g/100 g |
| Phenylalanine | 0.0146±0.0024 | g/100 g |
| Leucine | 0.0170±0.0027 | g/100 g |
| Proline | 0.0245±0.0039 | g/100 g |
| Serine | 0.0115±0.0019 | g/100 g |
| Valine | 0.0121±0.0019 | g/100 g |

### Example 3.

The following haircare products have been prepared, including the barley protein concentrate (shortly "BPC") obtained in Example 1.

### a) Shampoos comprising:

| **INCI** | **wt%** | **function** |
|---|---|---|
| AQUA | 70-90% | solvent |
| POLYQUATERNIUM-10 | 0.1-2% | conditioner |
| SODIUM C14-16 OLEFIN SULFONATE | 1-8% | surfactant |
| SODIUM COCOAMPHOACETATE | 1-8% | surfactant |
| LAURETH-5 CARBOXYLIC ACID | 1-8% | surfactant |
| PEG-18 GLYCERYL OLEATE/COCOATE | 1-5% | surfactant |
| PHENOXYETHANOL | 0.1-1% | preservative |
| BENZYL ALCOHOL | 0.1-1% | preservative |
| ETHYLHEXYLGLYCERIN | 0.1-1% | preservative |
| SODIUM GLUCEPTATE | 0.01-0.5% | chelating agent |
| **BPC** | 0.1-20% | active ingredient |
| PARFUM | 0.1-1% | perfume |
| PEG-40 HYDROGENATED CASTOR OIL | 0.1-1% | solvent |
| ETHOXYDIGLYCOL | 0.1-1% | solvent |
| CITRIC ACID | 0.1-1% | pH adjuster |
| PEG-120 METHYL GLUCOSE DIOLEATE | 0.01-1% | thickener |

### b) Conditioners comprising:

| **INCI** | **wt%** | **function** |
|---|---|---|
| AQUA | 60-90% | solvent |
| POLYQUATERNIUM-10 | 0.1-1% | conditioner |
| HYDROXYPROPYL STARCH PHOSPHATE | 0.1-4% | thickener |
| AQUA - CETRIMONIUM CHLORIDE | 0.1-4% | conditioner |
| DISTEAROYLETHYL HYDROXYETHYLMONIUM METHOSULFATE | 0.2-3% | conditioner |
| OLEA EUROPAEA OIL UNSAPONIFIABLES | 0.2-3% | emollient |
| OLEA EUROPAEA FRUIT OIL | 0.2-3% | emollient |
| HYDROGENATED VEGETABLE OIL | 0.2-3% | emollient |
| SORBITAN OLIVATE | 0.2-3% | emulsifier |
| CETEARYL OLIVATE | 0.2-3% | emollient |
| BUTYROSPERMUM PARKII BUTTER | 0.2-3% | emollient |
| CETEARYL ALCOHOL | 0.2-3% | emollient |
| DICOCOYLETHYL HYDROXYETHYLMONIUM METHOSULFATE | 0.2-3% | conditioner |
| PROPYLENE GLYCOL | 0.2-3% | humectant |
| BEHENYL ALCOHOL | 0.2-3% | emollient |
| GUAR HYDROXYPROPYLTRIMONIUM CHLORIDE | 0.2-3% | conditioner |
| HYDROXYPROPYLTRIMONIUM HYDROLYZED WHEAT PROTEIN | 0.2-3% | conditioner |
| SODIUM PCA | 0.2-3% | humectant |
| GLYCERIN | 0.2-3% | humectant |
| **BPC** | 0.1-20% | active ingredient |
| BETAINE | 0.2-3% | humectant |
| POLYQUATERNIUM-47 | 0.2-3% | conditioner |
| AMODIMETHICONE | 0.2-3% | antistatic |
| POTASSIUM SORBATE | 0.1-1% | preservative |
| BENZYL ALCOHOL CONS | 0.1-1% | preservative |
| SODIUM BENZOATE | 0.1-1% | preservative |
| SODIUM GLUCEPTATE | 0.1-1% | chelating agent |
| CITRIC ACID | 0.1-1% | pH adjuster |
| PARFUM | 0.1-1% | perfume |

### c) Masks comprising:

| **INCI** | **wt%** | **function** |
|---|---|---|
| AQUA | 60-90% | solvent |
| BEHENTRIMONIUM CHLORIDE | 0.2-6% | emulsifier |
| BUTYROSPERMUM PARKII BUTTER | 0.2-2% | emollient |
| GLYCERYL LINOLENATE | 0.2-2% | emollient |
| GLYCERYL LINOLEATE | 0.2-2% | emollient |
| GLYCERYL OLEATE | 0.2-2% | emollient |
| CETEARYL ALCOHOL | 2-8% | emollient |
| CETYL ESTERS | 2-5% | emollient |
| QUATERNIUM-87 | 0.5-5% | conditioner |
| PROPYLENE GLYCOL | 0.5-5% | humectant |
| AMODIMETHICONE | 0.5-5% | antistatic |
| ISOSTEARYL NEOPENTANOATE | 0.5-5% | emollient |
| POTASSIUM SORBATE | 0.1-1% | preservative |
| BENZYL ALCOHOL | 0.1-1% | preservative |
| SODIUM BENZOATE | 0.1-1% | preservative |
| SODIUM GLUCEPTATE | 0.1-1% | chelating agent |
| **BPC** | 0.1-20% | active ingredient |
| CITRIC ACID | 0.1-1% | pH adjuster |
| PARFUM | 0.1-1% | perfume |

### d) Leave-in creams comprising:

| **INCI** | **wt%** | **function** |
|---|---|---|
| AQUA | 60-90% | solvent |
| CITRIC ACID | 0.1-1% | pH adjuster |
| CETRIMONIUM CHLORIDE | 0.1-5% | emulsifier |
| CETYL ALCOHOL | 0.1-5% | emollient |
| OLETH-10 | 0.1-5% | emulsifier |
| SORBITAN OLEATE | 0.1-5% | emulsifier |
| DICOCOYLETHYL HYDROXYETHYLMONIUM METHOSULFATE | 0.1-5% | conditioner |
| PROPYLENE GLYCOL | 0.1-5% | humectant |
| DICAPRYLYL ETHER | 0.1-5% | emollient |
| CETEARYL ALCOHOL | 0.1-5% | emollient |
| BUTYROSPERMUM PARKII BUTTER | 0.1-5% | emollient |
| DIMETHICONE | 0.1-5% | emollient |
| PEG/PPG-14/4 DIMETHICONE | 0.1-5% | emulsifier |
| SORBITOL | 0.1-5% | humectant |
| BENZYL ALCOHOL CONS | 0.1-5% | preservative |
| SODIUM BENZOATE | 0.1-5% | preservative |
| POTASSIUM SORBATE | 0.1-5% | preservative |
| SODIUM GLUCEPTATE | 0.1-5% | chelating agent |
| **BPC** | 0.1-20% | active ingredient |
| AMODIMETHICONE | 0.1-5% | antistatic |
| PARFUM | 0.1-5% | perfume |

### e) Hairsprays comprising:

| **INCI** | **wt%** | **function** |
|---|---|---|
| ALCOHOL DENAT. | 50-90% | solvent |
| AMINOMETHYL PROPANOL | 0.01-0.1% | pH adjuster |
| ACRYLATES COPOLYMER | 0.2-15 % | fixative |
| AQUA | 0.1-10 % | solvent |
| **BPC** | 0.1-20%% | active ingredient |
| PARFUM | 0.1 - 0.9 | perfume |

### f) Oxidative hair dyes comprising:

| **INCI** | **wt%** | **function** |
|---|---|---|
| SORBITAN STEARATE | 0.5 - 5% | emulsifier |
| GLYCERYL ISOSTEARATE | 0.5 - 5% | emulsifier |
| COCOS NUCIFERA OIL | 0.5 - 5% | emollient |
| POLYSORBATE 60 | 0.5 - 9% | emulsifier |
| LAURETH-3 | 0.5 - 9% | emulsifier |
| PETROLATUM | 0.5 - 9% | emollient |
| OLEIC ACID | 0.5 - 9% | emulsifier |
| CERA ALBA | 0.5 - 9% | emollient |
| CETEARYL ALCOHOL | 1.5 - 15% | emollient |
| SORBITAN OLEATE | 0.2 - 2% | emulsifier |
| HYDROGENATED CASTOR OIL | 0.2 - 2% | emulsifier |
| CETEARETH-20 | 0.2 - 2% | surfactant |
| STEARIC ACID | 0.2 - 2% | emulsifier |
| PALMITIC ACID | 0.2 - 2% | emulsifier |
| POLYQUATERNIUM-22 | 0.1 - 2% | antistatic |
| ETIDRONIC ACID | 0.01 - 0.5% | chelating agent |
| PROPYLENE GLYCOL | 0.5 - 5% | emollient |
| TETRASODIUM EDTA | 0.01 - 0.5% | chelating agent |
| COCAMIDOPROPYL BETAINE | 0.5 - 6% | surfactant |
| AMMONIA (in combination or substituted with: ETHANOLAMINE, AMINOMETHYL PROPANOL, AMMONIUM BICARBONATE, DIMETHYLGLUCAMINE) | 0.5 - 10% | pH adjuster |
| **BPC** | 0.1-20% | active ingredient |
| AQUA | 60 - 80% | solvent |
| PARFUM | 0.1 - 0.9% | perfume |
| TOLUENE-2.5-DIAMINE SULFATE | 0.01 - 1% | colorant |
| p-AMINOPHENOL | 0.01 - 1% | colorant |
| p-METHYLAMINOPHENOL SULFATE | 0.01 - 1% | colorant |
| m-AMINOPHENOL | 0.01 - 1% | colorant |
| 1-NAPHTHOL | 0.01 - 1% | colorant |
| 4-CHLORORESORCINOL | 0.01 - 1% | colorant |
| 4-AMINO-2-HYDROXYTOLUENE | 0.01 - 1% | colorant |
| 2-METHYLRESORCINOL | 0.01 - 1% | colorant |
| PHENYL METHYL PYRAZOLONE | 0.01 - 1% | colorant |
| SODIUM HYDROSULFITE | 0.01 - 1% | antioxidant |
| ERYTHORBIC ACID | 0.01 - 1% | antioxidant |
| SODIUM SULFITE | 0.01 - 1% | antioxidant |

### g) Decolouring bases comprising:

| **INCI** | **wt%** | **c** |
|---|---|---|
| SODIUM SILICATE | 20-50% | pH adjuster |
| AMMONIUM PERSULFATE | 20-50% | oxidant |
| POTASSIUM PERSULFATE | 13-20% | oxidant |
| SODIUM METASILICATE | 0.2-5% | chelating agent |
| SODIUM STEARATE | 0.2-5% | surfactant |
| CYAMOPSIS TETRAGONOLOBA GUM | 0.2-5% | ligand |
| SODIUM LAURYL SULFATE | 0.2-5% | surfactant |
| MAGNESIUM OXIDE | 0.2-5% | pH adjuster |
| CYCLODEXTRIN | 0.2-5% | chelating agent |
| MAGNESIUM STEARATE | 0.2-5% | antiagglomerant |
| PARAFFINUM LIQUIDUM | 0.2-5% | emollient |
| XANTHAN GUM | 0.2-5% | ligand |
| **BPC** | 0.1-20% | active ingredient |

### h) Serums comprising:

| **INCI** | **%** | **function** |
|---|---|---|
| **BPC** | 0.1-20% | active ingredient |
| AQUA | 60-90% | solvent |
| HYDROXYPROPYL GUAR | 0.1-1% | thickener |
| GLICERINE | 0.1-10% | emollient |
| PROPANEDIOL | 0.1-10% | emollient |
| GUAR HYDROXYPROPYLTRIMONIUM CHLORIDE | 0.1-1% | conditioner |
| BENZOIC ACID | 0.1-1% | preservative |
| DEHYDROACETIC ACID | 0.1-1% | preservative |
| PHENOXYETHANOL | 0.1-1% | preservative |
| SODIUM BENZOATE | 0.1-1% | preservative |
| POTASSIUM SORBATE | 0.1-1% | preservative |
| BENZYL ALCOHOL | 0.1-1% | preservative |
| CETRIMONIUM CHLORIDE | 0.1-1% | surfactant |
| PARFUM | 0.1-0.9% | perfume |

### Example 4.

The efficacy of BPC was tested on human hair strands that have undergone a colouring process with oxidation colour.

The coloured strands were treated by using the products of the invention, having the compositions of the Example 3, while following the procedure below:

| **Product / Operation** | **BPC (wt%)** | **Application time** |
|---|---|---|
| Shampoo | 1 wt% | 5 minutes |
| Rinse with water | - | 2 minutes |
| Mask | 2.5 wt% | 10 - 15 minutes |
| Rinse with water | - | 2 minutes |
| Serum | 5 wt% | 10 - 15 minutes |
| Rinse with water | - | 2 minutes |
| Leave-In cream | 2.5 wt% | 2 minutes |
| Drying | - | 5 minutes |

A control test was also carried out, where the same haircare products above, but free of BPC, were used as comparative products, while following the same procedure.

### Microscope Analysis

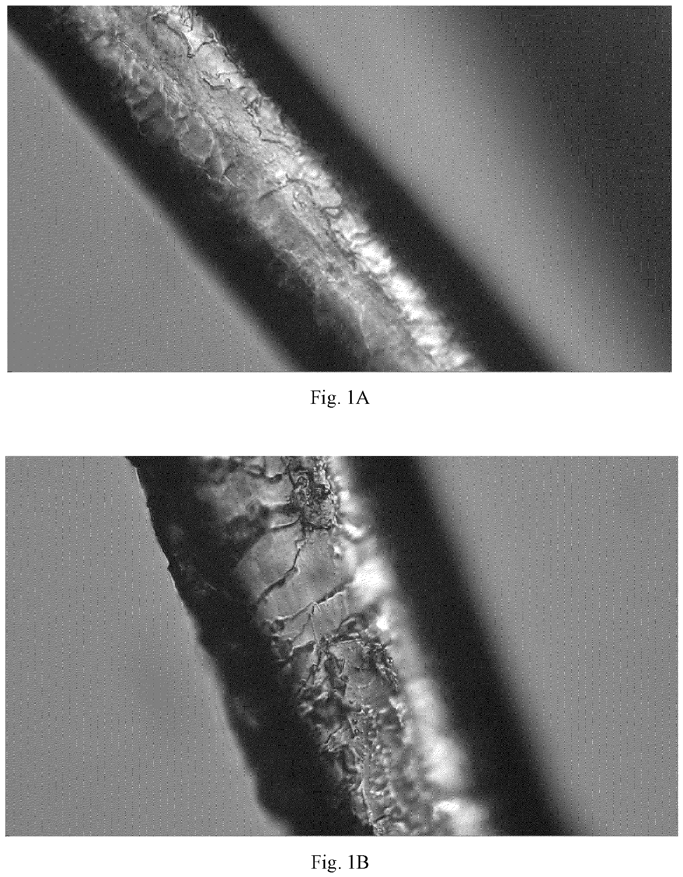

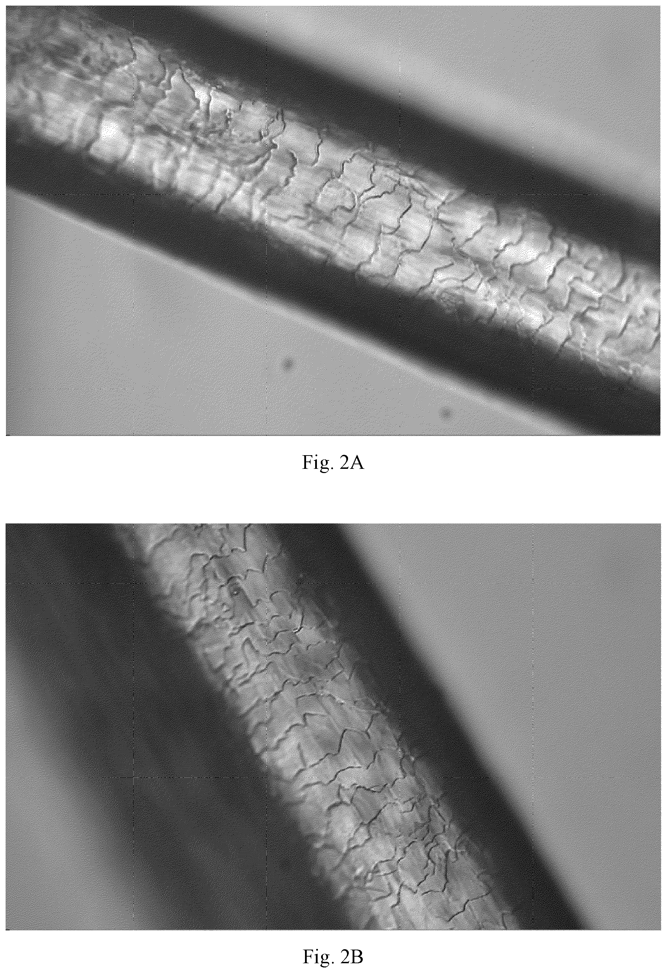

The optical microscope images of the two different treatments are shown in the Figures. In particular, Figg. 1A and 1B show a 40x magnification of damaged hair treated with comparative products, where it is clear that no reparation occurred on their surface. Conversely, in Figg. 2A and 2B, a 40x magnification of damaged hair treated with the haircare products of the present invention is shown, where it is evident the repairing efficacy, as the cuticles appear to be homogenously closed.

### Example 5.

### Hair breakage test

### Materials and methods

In order to perform the test, 10 locks of natural hair, brown background, with an average weight of 7 g (± 0.5 g) were prepared.

The protocol chosen for the test is reported below:
- 5 locks were treated with *mixture 1*: 15g of a decolourant and 30g of an oxidant
- 5 locks were treated with *mixture 2*: 15g of a decolourant and 30g of an oxidant added with 5 grams of serum according to Example 4.

Application time: 45 minutes on a hair straightener set at 35°C

Procedure followed:
- Rinse, wash with shampoo (see Example 4), blow dry
- Repeat the decolouring procedure for two more cycles
- Weight of the lock
- Cycle of 10 combings
- Recovery of broken hair on black background
- Evaluation of the weight of the broken hair
- Sensory evaluation of the hair: volume, mass, conditioning, decolouring tone, flexibility, combability, shine

### Results

Below is the table with the percentage weights of the broken hair (reported as a percentage of the weight of the broken hair compared to the weight of the lock before combing):

### 1) Hair locks treated with mixture 1

| | **Weight loss %** |
|---|---|
| lock 1 | 0.315 |
| lock 2 | 0.215 |
| lock 3 | 0.255 |
| lock 4 | 0.269 |
| lock 5 | 0.361 |
| **Average value** | **0.283** |

### 2) Hair locks treated with mixture 2

| | **Weight loss %** |
|---|---|
| lock 1 | 0.00265 |
| lock 2 | 0.00275 |
| lock 3 | 0.00312 |
| lock 4 | 0.00584 |
| lock 5 | 0.00648 |
| **Average value** | **0.00416** |

### Discussion

The analysis of both individual and average values shows a highly significant decrease in broken hair in the treatment group 2.

In fact, it should be appreciated that the hair locks treated with the mixture 2 feature a reduction of broken hair of 68 times than the hair locks treated with the mixture 1.

### Conclusion

The addition of BPC of the invention to haircare products is significantly effective in preventing and repairing damage to the hair structure, while making hair stronger and more resistant to breakage.

## Claims

1. A process for extracting a barley protein concentrate comprises the steps of:
i) providing a water suspension of 2-20wt% spent barley grains,
ii) adjusting the pH of the water suspension to basic, under stirring,
iii) heating the basic suspension to a temperature of 45-75°C and keeping under stirring for at least 1 hour, and
iv) filtering off the suspension and then cooling down the resulting filtrate to 15-25°C, where said filtrate is the barley protein concentrate.

2. The process of claim 1, wherein, in step i), spent barley grains are provided and then suspended in water to achieve a concentration of 5-15wt%.

3. The process of claim 1 or 2, wherein, in step iii), the basic suspension is heated to a temperature of 55-65°C, then kept under stirring for 1-8 hours.

4. The process of any one of claims 1 to 3, wherein, at the end of step iv), the barley protein concentrate is added with a preservative agent, preferably in a concentration of 0.1-5wt%, based on the concentrate weight.

5. The process of any one of claims 1 to 4, wherein, at the end of step iv), the barley protein concentrate is added with a rheological additive, preferably in a concentration of 0. 1-5wt%, based on the concentrate weight.

6. The process of any one of claims 1 to 5, wherein, at the end of step iv), the barley protein concentrate is added with an acidifier, preferably in a concentration of 0.01-0.5wt%, based on the concentrate weight, so as to adjust the pH of the barley protein concentrate to 3.5-6.5.

7. The process of any one of claims 1 to 6, wherein, step ii) is performed by applying ultrasonic technology.

8. The process of any one of claims 1 to 7, wherein, step iii) is performed by applying ultrasonic technology.

9. A barley protein concentrate obtained by the process of any one of claims 1 to 8, the concentrate having a protein content of 0.01-2.0 g/100 g of the concentrate, preferably 0.1-1.0 g/100 g, more preferably 0.1-0.5 g/100 g, as measured according to AOAC 992.23-1992(1998).

10. The barley protein concentrate of claim 9, further having a Total Kjeldahl Nitrogen (or TKN) content of 0.001-1.0 g/100 g of the concentrate, preferably 0.01-0.1 g/100 g.

11. The barley protein concentrate of claim 9 or 10, further having an amino acid content of 0.01-2.0 g/100 g of the concentrate, preferably 0.1-1.0 g/100 g, as measured according to ISO 13903:2005.

12. The barley protein concentrate of claim 11, wherein the amino acid is Aspartic acid, Glutamic acid, Alanine, Arginine, Phenylalanine, Leucine, Proline, Serine, Valine, or a mixture thereof.

13. A haircare product comprising 0.1-20wt% of the barley protein concentrate of any one of claims 9 to 12, based on the haircare product weight.

14. The haircare product of claim 13, in the form of lotion, milk, mousse, gel, cream, shampoo, conditioner, compress, mask, oil, emulsion o/w and emulsion w/o, silicone emulsion, multiple emulsion, microemulsion, hydroalcoholic solution, hydroglyceric solution, ointment, lipogel, paste, stick, cream-gel, spray, oxidative dyes, serum, decolouring base, or combination thereof, such as a kit of combined products.
